# EUROPÄISCHE PATENTSCHRIFT

(11) **EP 3 115 045 B1**
(45) Veröffentlichungstag und Bekanntmachung des Hinweises auf die Patenterteilung: **12.08.2020**
(21) Anmeldenummer: 16175305.8
(22) Anmeldetag: 26.08.2009
(51) Int. Cl.: A61K 31/135, A61K 31/155, A61K 31/357, A61K 31/381, A61K 31/4164, A61K 31/443, A61K 31/445, A61K 31/4743, A61K 31/496, A61K 31/506, A61P 13/02, A61P 35/00, A61P 29/00, A61G 9/00, A61K 6/00, A61K 8/00, A61Q 11/02, A61Q 17/02, A23G 9/00

(54) **NACHWEIS UND VERWENDUNG NIEDERMOLEKULARER MODULATOREN DES KÄLTE-MENTHOL-REZEPTORS TRPM8**
DETECTION AND USE OF LOW MOLECULAR WEIGHT MODULATORS OF THE COLD MENTHOL RECEPTOR TRPM8
VERIFICATION ET UTILISATION DE MODULATEURS SOUS-MOLECULAIRES DU RECEPTEUR DE FROID ET DE MENTHOL TRPM8

(30) Priorität: 26.08.2008 EP 08162997
(43) Veröffentlichungstag der Anmeldung: 11.01.2017
(62) Teilanmeldung aus: 09782234.0
(73) Patentinhaber: BASF SE, 67056 Ludwigshafen am Rhein (DE)
(72) Erfinder: SUBKOWSKI, Thomas, 68526 Ladenburg (DE); BOLLSCHWEILER, Claus, 69118 Heidelberg (DE); WITTENBERG, Jens, 67117 Limburgerhof (DE); KROHN, Michael, 64653 Lorsch (DE); ZINKE, Holger, 64673 Zwingenberg (DE)

(56) Entgegenhaltungen:
- WO-A-2004/026840
- WO-A-2007/017093
- WO-A-2008/015403
- WO-A1-98/58905
- WO-A1-2005/099711
- WO-A1-2007/080109
- WO-A2-2006/097337
- WO-A2-2009/064388
- DE-A1- 10 234 876
- JP-A- 2000 302 760
- US-A1- 2005 084 447
- US-A1- 2005 203 159
- US-A1- 2005 250 836
- INGALE SANJAY J ET AL: "Synthesis and biological evaluation of some 4-substituted quinoxalinones", ASIAN JOURNAL OF CHEMISTRY, CHEMIC PUBLISHING, SAHIBADAD, IN, Bd. 19, Nr. 5, 31. Oktober 2007 (2007-10-31), Seiten 3797-3802, XP009193906, ISSN: 0970-7077
- DEMIRAYAK SEREF ET AL: "SYNTHESIS AND ANALGESIC ACTIVITIES OF SOME 2-(BENZAZOLYLACETYL)AMINO-3-ETHOXYCARBONYL THIOPHENE DERIVATIVES", PHOSPHORUS, SULFUR AND SILICON AND THE RELATED ELEMENTS, TAYLOR & FRANCIS INC, US, Bd. 180, 1. Januar 2005 (2005-01-01), Seiten 1841-1848, XP009072026, ISSN: 1042-6507, DOI: 10.1080/104265090889503
- PATCH R J ET AL: "Identification of 2-acylaminothiophene-3-carboxamides as potent inhibitors of FLT3", BIOORGANIC & MEDICINAL CHEMISTRY LETTERS, PERGAMON, AMSTERDAM, NL, Bd. 16, Nr. 12, 15. Juni 2006 (2006-06-15) , Seiten 3282-3286, XP027965597, ISSN: 0960-894X [gefunden am 2006-06-15]
- COOKE A ET AL: "Water-soluble propofol analogues with intravenous anaesthetic activity", BIOORGANIC & MEDICINAL CHEMISTRY LETTERS, PERGAMON, AMSTERDAM, NL, Bd. 11, Nr. 7, 9. April 2001 (2001-04-09), Seiten 927-930, XP004232526, ISSN: 0960-894X, DOI: 10.1016/S0960-894X(01)00088-9

### SEQUENCE LISTING

<110> BASF SE
<120> Nachweis und Verwendung niedermolekularer Modulatoren des Kälte-Menthol-Rezeptors TRPM8
<130> M/49197-PCT
<160> 2
<170> PatentIn version 3.3
<210> 1
   <211> 5621
   <212> DNA
   <213> Homo sapiens
<400> 1
   aagaaaatcc tgcttgacaa aaaccgtcac ttaggaaaag atgtcctttc gggcagccag     60
   gctcagcatg aggaacagaa ggaatgacac tctggacagc acccggaccc tgtactccag    120
   cgcgtctcgg agcacagact tgtcttacag tgaaagcgac ttggtgaatt ttattcaagc    180
   aaattttaag aaacgagaat gtgtcttctt taccaaagat tccaaggcca cggagaatgt    240
   gtgcaagtgt ggctatgccc agagccagca catggaaggc acccagatca accaaagtga    300
   gaaatggaac tacaagaaac acaccaagga atttcctacc gacgcctttg gggatattca     360
   gtttgagaca ctggggaaga aagggaagta tatacgtctg tcctgcgaca cggacgcgga    420
   aatcctttac gagctgctga cccagcactg gcacctgaaa acacccaacc tggtcatttc    480
   tgtgaccggg ggcgccaaga acttcgccct gaagccgcgc atgcgcaaga tcttcagccg    540
   gctcatctac atcgcgcagt ccaaaggtgc ttggattctc acgggaggca cccattatgg    600
   cctgatgaag tacatcgggg aggtggtgag agataacacc atcagcagga gttcagagga    660
   gaatattgtg gccattggca tagcagcttg gggcatggtc tccaaccggg acaccctcat     720
   caggaattgc gatgctgagg gctatttttt agcccagtac cttatggatg acttcacaag    780
   agatccactg tatatcctgg acaacaacca cacacatttg ctgctcgtgg acaatggctg    840
   tcatggacat cccactgtcg aagcaaagct ccggaatcag ctagagaagt atatctctga    900
   gcgcactatt caagattcca actatggtgg caagatcccc attgtgtgtt ttgcccaagg    960
   aggtggaaaa gagactttga aagccatcaa tacctccatc aaaaataaaa ttccttgtgt   1020
   ggtggtggaa ggctcgggcc agatcgctga tgtgatcgct agcctggtgg aggtggagga   1080
   tgccctgaca tcttctgccg tcaaggagaa gctggtgcgc tttttacccc gcacggtgtc   1140
   ccggctgcct gaggaggaga ctgagagttg gatcaaatgg ctcaaagaaa ttctcgaatg   1200
   ttctcaccta ttaacagtta ttaaaatgga agaagctggg gatgaaattg tgagcaatgc   1260
   catctcctac gctctataca aagccttcag caccagtgag caagacaagg ataactggaa   1320
   tgggcagctg aagcttctgc tggagtggaa ccagctggac ttagccaatg atgagatttt   1380
   caccaatgac cgccgatggg agtctgctga ccttcaagaa gtcatgttta cggctctcat   1440
   aaaggacaga cccaagtttg tccgcctctt tctggagaat ggcttgaacc tacggaagtt   1500
   tctcacccat gatgtcctca ctgaactctt ctccaaccac ttcagcacgc ttgtgtaccg   1560
   gaatctgcag atcgccaaga attcctataa tgatgccctc ctcacgtttg tctggaaact   1620
   ggttgcgaac ttccgaagag gcttccggaa ggaagacaga aatggccggg acgagatgga   1680
   catagaactc cacgacgtgt ctcctattac tcggcacccc ctgcaagctc tcttcatctg   1740
   ggccattctt cagaataaga aggaactctc caaagtcatt tgggagcaga ccaggggctg   1800
   cactctggca gccctgggag ccagcaagct tctgaagact ctggccaaag tgaagaacga   1860
   catcaatgct gctggggagt ccgaggagct ggctaatgag tacgagaccc gggctgttga   1920
   gctgttcact gagtgttaca gcagcgatga agacttggca gaacagctgc tggtctattc   1980
   ctgtgaagct tggggtggaa gcaactgtct ggagctggcg gtggaggcca cagaccagca   2040
   tttcatcgcc cagcctgggg tccagaattt tctttctaag caatggtatg gagagatttc   2100
   ccgagacacc aagaactgga agattatcct gtgtctgttt attataccct tggtgggctg   2160
   tggctttgta tcatttagga agaaacctgt cgacaagcac aagaagctgc tttggtacta   2220
   tgtggcgttc ttcacctccc ccttcgtggt cttctcctgg aatgtggtct tctacatcgc   2280
   cttcctcctg ctgtttgcct acgtgctgct catggatttc cattcggtgc cacacccccc   2340
   cgagctggtc ctgtactcgc tggtctttgt cctcttctgt gatgaagtga gacagtggta   2400
   cgtaaatggg gtgaattatt ttactgacct gtggaatgtg atggacacgc tggggctttt   2460
   ttacttcata gcaggaattg tatttcggct ccactcttct aataaaagct ctttgtattc   2520
   tggacgagtc attttctgtc tggactacat tattttcact ctaagattga tccacatttt   2580
   tactgtaagc agaaacttag gacccaagat tataatgctg cagaggatgc tgatcgatgt   2640
   gttcttcttc ctgttcctct ttgcggtgtg gatggtggcc tttggcgtgg ccaggcaagg   2700
   gatccttagg cagaatgagc agcgctggag gtggatattc cgttcggtca tctacgagcc   2760
   ctacctggcc atgttcggcc aggtgcccag tgacgtggat ggtaccacgt atgactttgc   2820
   ccactgcacc ttcactggga atgagtccaa gccactgtgt gtggagctgg atgagcacaa   2880
   cctgccccgg ttccccgagt ggatcaccat ccccctggtg tgcatctaca tgttatccac   2940
   caacatcctg ctggtcaacc tgctggtcgc catgtttggc tacacggtgg gcaccgtcca   3000
   ggagaacaat gaccaggtct ggaagttcca gaggtacttc ctggtgcagg agtactgcag   3060
   ccgcctcaat atccccttcc ccttcatcgt cttcgcttac ttctacatgg tggtgaagaa   3120
   gtgcttcaag tgttgctgca aggagaaaaa catggagtct tctgtctgct gtttcaaaaa   3180
   tgaagacaat gagactctgg catgggaggg tgtcatgaag gaaaactacc ttgtcaagat   3240
   caacacaaaa gccaacgaca cctcagagga aatgaggcat cgatttagac aactggatac 3300
   aaagcttaat gatctcaagg gtcttctgaa agagattgct aataaaatca aataaaactg 3360
   tatgaactct aatggagaaa aatctaatta tagcaagatc atattaagga atgctgatga 3420
   acaattttgc tatcgactac taaatgagag attttcagac ccctgggtac atggtggatg 3480
   attttaaatc accctagtgt gctgagacct tgagaataaa gtgtgtgatt ggtttcatac 3540
   ttgaagacgg atataaagga agaatatttc ctttatgtgt ttctccagaa tggtgcctgt 3600
   ttctctctgt gtctcaatgc ctgggactgg aggttgatag tttaagtgtg ttcttaccgc 3660
   ctcctttttc ctttaatctt atttttgatg aacacatata taggagaaca tctatcctat 3720
   gaataagaac ctggtcatgc tttactcctg tattgttatt ttgttcattt ccaattgatt 3780
   ctctactttt cccttttttg tattatgtga ctaattagtt ggcatattgt taaaagtctc 3840
   tcaaattagg ccagattcta aaacatgctg cagcaagagg accccgctct cttcaggaaa 3900
   agtgttttca tttctcagga tgcttcttac ctgtcagagg aggtgacaag gcagtctctt 3960
   gctctcttgg actcaccagg ctcctattga aggaaccacc cccattccta aatatgtgaa 4020
   aagtcgccca aaatgcaacc ttgaaaggca ctactgactt tgttcttatt ggatactcct 4080
   cttattattt ttccattaaa aataatagct ggctattata gaaaatttag accatacaga 4140
   gatgtagaaa gaacataaat tgtccccatt accttaaggt aatcactgct aacaatttct 4200
   ggatggtttt tcaagtctat tttttttcta tgtatgtctc aattctcttt caaaatttta 4260
   cagaatgtta tcatactaca tatatacttt ttatgtaagc tttttcactt agtattttat 4320
   caaatatgtt tttattatat tcatagcctt cttaaacatt atatcaataa ttgcataata 4380
   ggcaacctct agcgattacc ataattttgc tcattgaagg ctatctccag ttgatcattg 4440
   ggatgagcat ctttgtgcat gaatcctatt gctgtatttg ggaaaatttt ccaaggttag 4500
   attccaataa atatctattt attattaaat attaaaatat ctatttatta ttaaaaccat 4560
   ttataaggct ttttcataaa tgtatagcaa ataggaatta ttaacttgag cataagatat 4620
   gagatacatg aacctgaact attaaaataa aatattatat ttaaccctta gtttaagaag 4680
   aagtcaatat gcttatttaa atattatgga tggtgggcag atcacttgag gtcaggagtt 4740
   cgagaccagc ctggccaaca tggcaaaacc acatctctac taaaaataaa aaaattagct 4800
   gggtgtggtg gtgcactcct gtaatcccag ctactcagaa ggctgaggta caagaattgc 4860
   tggaacctgg gaggcggagg ttgcagtgaa ccaagattgc accactgcac tccagccggg 4920
   gtgacagagt gagactccga ctgaaaataa ataaataaat aaataaataa ataaataaat 4980
   attatggatg gtgaagggaa tggtatagaa ttggagagat tatcttactg aacacctgta 5040
   gtcccagctt tctctggaag tggtcgtatt tgagcaggat gtgcacaagg caattgaaat 5100
   gcccataatt agtttctcag ctttgaatac actataaact cactggctga aggaggaaat   5160
   tttagaagga agctactaaa agatctaatt tgaaaaacta caaaagcatt aactaaaaaa   5220
   gtttattttc cttttgtctg ggcagtagtg aaaataacta ctcacaacat tcactatgtt   5280
   tgcaaggaat taacacaaat aaaagatgcc tttttactta aacaccaaga cagaaaactt   5340
   gcccaatact gagaagcaac ttgcattaga gagggaactg ttaaatgttt tcaacccagt   5400
   tcatctggtg gatgtttttg caggttactc tgagaatttt gcttatgaaa aatcattatt   5460
   tttagtgtag ttcacaataa tgtattgaac atacttctaa tcaaaggtgc tatgtccttg   5520
   tgtatggtac taaatgtgtc ctgtgtactt ttgcacaact gagaatcctg cagcttggtt   5580
   taatgagtgt gttcatgaaa taaataatgg aggaattgtc a                        5621
<210> 2
   <211> 1104
   <212> **PRT**
   <213> Homo sapiens
<400> 2
   Met Ser Phe Arg Ala Ala Arg Leu Ser Met Arg Asn Arg Arg Asn Asp
   1               5                   10                  15
   Thr Leu Asp Ser Thr Arg Thr Leu Tyr Ser Ser Ala Ser Arg Ser Thr
               20                  25                  30
   Asp Leu Ser Tyr Ser Glu Ser Asp Leu Val Asn Phe Ile Gln Ala Asn
           35                  40                  45
   Phe Lys Lys Arg Glu Cys Val Phe Phe Thr Lys Asp Ser Lys Ala Thr
       50                  55                  60
   Glu Asn Val Cys Lys Cys Gly Tyr Ala Gln Ser Gln His Met Glu Gly
   65                  70                  75                  80
   Thr Gln Ile Asn Gln Ser Glu Lys Trp Asn Tyr Lys Lys His Thr Lys
                   85                  90                  95
   Glu Phe Pro Thr Asp Ala Phe Gly Asp Ile Gln Phe Glu Thr Leu Gly
               100                 105                 110
   Lys Lys Gly Lys Tyr Ile Arg Leu Ser Cys Asp Thr Asp Ala Glu Ile
           115                 120                 125
   Leu Tyr Glu Leu Leu Thr Gln His Trp His Leu Lys Thr Pro Asn Leu
       130                 135                 140
   Val Ile Ser Val Thr Gly Gly Ala Lys Asn Phe Ala Leu Lys Pro Arg
   145 150 155 160
   Met Arg Lys Ile Phe Ser Arg Leu Ile Tyr Ile Ala Gln Ser Lys Gly
   165 170 175
   Ala Trp Ile Leu Thr Gly Gly Thr His Tyr Gly Leu Met Lys Tyr Ile
   180 185 190
   Gly Glu Val Val Arg Asp Asn Thr Ile Ser Arg Ser Ser Glu Glu Asn
   195 200 205
   Ile Val Ala Ile Gly Ile Ala Ala Trp Gly Met Val Ser Asn Arg Asp
   210 215 220
   Thr Leu Ile Arg Asn Cys Asp Ala Glu Gly Tyr Phe Leu Ala Gln Tyr
   225 230 235 240
   Leu Met Asp Asp Phe Thr Arg Asp Pro Leu Tyr Ile Leu Asp Asn Asn
   245 250 255
   His Thr His Leu Leu Leu Val Asp Asn Gly Cys His Gly His Pro Thr
   260 265 270
   Val Glu Ala Lys Leu Arg Asn Gln Leu Glu Lys Tyr Ile Ser Glu Arg
   275 280 285
   Thr Ile Gln Asp Ser Asn Tyr Gly Gly Lys Ile Pro Ile Val Cys Phe
   290 295 300
   Ala Gln Gly Gly Gly Lys Glu Thr Leu Lys Ala Ile Asn Thr Ser Ile
   305 310 315 320
   Lys Asn Lys Ile Pro Cys Val Val Val Glu Gly Ser Gly Gln Ile Ala
   325 330 335
   Asp Val Ile Ala Ser Leu Val Glu Val Glu Asp Ala Leu Thr Ser Ser
   340 345 350
   Ala Val Lys Glu Lys Leu Val Arg Phe Leu Pro Arg Thr Val Ser Arg
   355 360 365
   Leu Pro Glu Glu Glu Thr Glu Ser Trp Ile Lys Trp Leu Lys Glu Ile
   370 375 380
   Leu Glu Cys Ser His Leu Leu Thr Val Ile Lys Met Glu Glu Ala Gly
   385 390 395 400
   Asp Glu Ile Val Ser Asn Ala Ile Ser Tyr Ala Leu Tyr Lys Ala Phe
   405 410 415
   Ser Thr Ser Glu Gln Asp Lys Asp Asn Trp Asn Gly Gln Leu Lys Leu
   420 425 430
   Leu Leu Glu Trp Asn Gln Leu Asp Leu Ala Asn Asp Glu Ile Phe Thr
   435 440 445
   Asn Asp Arg Arg Trp Glu Ser Ala Asp Leu Gln Glu Val Met Phe Thr
   450 455 460
   Ala Leu Ile Lys Asp Arg Pro Lys Phe Val Arg Leu Phe Leu Glu Asn
   465 470 475 480
   Gly Leu Asn Leu Arg Lys Phe Leu Thr His Asp Val Leu Thr Glu Leu
   485 490 495
   Phe Ser Asn His Phe Ser Thr Leu Val Tyr Arg Asn Leu Gln Ile Ala
   500 505 510
   Lys Asn Ser Tyr Asn Asp Ala Leu Leu Thr Phe Val Trp Lys Leu Val
   515 520 525
   Ala Asn Phe Arg Arg Gly Phe Arg Lys Glu Asp Arg Asn Gly Arg Asp
   530 535 540
   Glu Met Asp Ile Glu Leu His Asp Val Ser Pro Ile Thr Arg His Pro
   545 550 555 560
   Leu Gln Ala Leu Phe Ile Trp Ala Ile Leu Gln Asn Lys Lys Glu Leu
   565 570 575
   Ser Lys Val Ile Trp Glu Gln Thr Arg Gly Cys Thr Leu Ala Ala Leu
   580 585 590
   Gly Ala Ser Lys Leu Leu Lys Thr Leu Ala Lys Val Lys Asn Asp Ile
   595 600 605
   Asn Ala Ala Gly Glu Ser Glu Glu Leu Ala Asn Glu Tyr Glu Thr Arg
   610 615 620
   Ala Val Glu Leu Phe Thr Glu Cys Tyr Ser Ser Asp Glu Asp Leu Ala
   625 630 635 640
   Glu Gln Leu Leu Val Tyr Ser Cys Glu Ala Trp Gly Gly Ser Asn Cys
   645 650 655
   Leu Glu Leu Ala Val Glu Ala Thr Asp Gln His Phe Ile Ala Gln Pro
               660                 665                 670
   Gly Val Gln Asn Phe Leu Ser Lys Gln Trp Tyr Gly Glu Ile Ser Arg
           675                 680                 685
   Asp Thr Lys Asn Trp Lys Ile Ile Leu Cys Leu Phe Ile Ile Pro Leu
       690                 695                 700
   Val Gly Cys Gly Phe Val Ser Phe Arg Lys Lys Pro Val Asp Lys His
   705                 710                 715                 720
   Lys Lys Leu Leu Trp Tyr Tyr Val Ala Phe Phe Thr Ser Pro Phe Val
                   725                 730                 735
   Val Phe Ser Trp Asn Val Val Phe Tyr Ile Ala Phe Leu Leu Leu Phe
               740                 745                 750
   Ala Tyr Val Leu Leu Met Asp Phe His Ser Val Pro His Pro Pro Glu
           755                 760                 765
   Leu Val Leu Tyr Ser Leu Val Phe Val Leu Phe Cys Asp Glu Val Arg
       770                 775                 780
   Gln Trp Tyr Val Asn Gly Val Asn Tyr Phe Thr Asp Leu Trp Asn Val
   785                 790                 795                 800
   Met Asp Thr Leu Gly Leu Phe Tyr Phe Ile Ala Gly Ile Val Phe Arg
                   805                 810                 815
   Leu His Ser Ser Asn Lys Ser Ser Leu Tyr Ser Gly Arg Val Ile Phe
               820                 825                 830
   Cys Leu Asp Tyr Ile Ile Phe Thr Leu Arg Leu Ile His Ile Phe Thr
           835                 840                 845
   Val Ser Arg Asn Leu Gly Pro Lys Ile Ile Met Leu Gln Arg Met Leu
       850                 855                 860
   Ile Asp Val Phe Phe Phe Leu Phe Leu Phe Ala Val Trp Met Val Ala
   865                 870                 875                 880
   Phe Gly Val Ala Arg Gln Gly Ile Leu Arg Gln Asn Glu Gln Arg Trp
                   885                 890                 895
   Arg Trp Ile Phe Arg Ser Val Ile Tyr Glu Pro Tyr Leu Ala Met Phe
               900                 905                 910
   Gly Gln Val Pro Ser Asp Val Asp Gly Thr Thr Tyr Asp Phe Ala His
           915                 920                 925
   Cys Thr Phe Thr Gly Asn Glu Ser Lys Pro Leu Cys Val Glu Leu Asp
       930                 935                 940
   Glu His Asn Leu Pro Arg Phe Pro Glu Trp Ile Thr Ile Pro Leu Val
   945                 950                 955                 960
   Cys Ile Tyr Met Leu Ser Thr Asn Ile Leu Leu Val Asn Leu Leu Val
                   965                 970                 975
   Ala Met Phe Gly Tyr Thr Val Gly Thr Val Gln Glu Asn Asn Asp Gln
               980                 985                 990
   Val Trp Lys Phe Gln Arg Tyr Phe  Leu Val Gln Glu Tyr  Cys Ser Arg
           995                 1000                 1005
   Leu Asn  Ile Pro Phe Pro Phe  Ile Val Phe Ala Tyr  Phe Tyr Met
       1010                 1015                 1020
   Val Val  Lys Lys Cys Phe Lys  Cys Cys Cys Lys Glu  Lys Asn Met
       1025                 1030                 1035
   Glu Ser  Ser Val Cys Cys Phe  Lys Asn Glu Asp Asn  Glu Thr Leu
       1040                 1045                 1050
   Ala Trp  Glu Gly Val Met Lys  Glu Asn Tyr Leu Val  Lys Ile Asn
       1055                 1060                 1065
   Thr Lys  Ala Asn Asp Thr Ser  Glu Glu Met Arg His  Arg Phe Arg
       1070                 1075                 1080
   Gln Leu  Asp Thr Lys Leu Asn  Asp Leu Lys Gly Leu  Leu Lys Glu
       1085                 1090                 1095
   Ile Ala  Asn Lys Ile Lys
       1100

## Beschreibung

Die Erfindung betrifft neuartige Modulatoren des Kälte-Menthol-Rezeptors TRPM8, Verfahren zur Modulation des TRPM8-Rezeptors unter Verwendung dieser Modulatoren; die Verwendung der Modulatoren zur Induktion von Kältegefühl; sowie die unter Verwendung dieser Modulatoren hergestellten Gegenstände und Mittel.

### Hintergrund der Erfindung

Der Kälte-Menthol-Rezeptor TRPM8 (auch bezeichnet als Cold-Membrane Receptor (CMR)1) gehört zur Familie der "Transient Receptor Potential Ion Channels", wird spezifisch in einer speziellen Gruppe von Neuronen exprimiert und bildet in der Zellmembran Poren aus (jeweils 4 Einheiten lagern sich dabei zu einem Tetramer zusammen), die selektiv Ca²⁺ Ionen passieren lassen. Das Protein weist 6 Transmembrandomänen auf und einen cytoplasmatischen C- sowie N-Terminus. Durch niedrige Temperaturen (bevorzugt 10-25°C) wird dieser Rezeptor stimuliert, es kommt zu einer Signaltransduktion, die vom Nervensystem als Kältegefühl interpretiert wird. Der Rezeptor ist erstmals 2002 als Kälterezeptor in mehreren Publikationen beschrieben worden (Peier AM et al, .A TRP channel that senses cold stimuli and menthol.Cell. 2002 Mar 8;108(5):705-15; McKemy DD et al. Identification of a cold receptor reveals a general role for TRP channels in thermosensation. Nature. 2002 Mar 7;416(6876):52-8; Zuker CS. Neurobiology: a cool ion channel.Nature. 2002 Mar 7;416(6876):27-8)

Kühlende Verbindungen, wie z.B. Menthol, spielen bereits seit langem eine wichtige Rolle in der Geschmacks- und Riechstoffindustrie, um eine Assoziation mit Frische und Sauberkeit zu erzeugen. Für die Verbindung Menthol ist gezeigt worden, dass sie als ein natürlicher Modulator des Rezeptors TRPM8 wirkt (McKemy D.D., Molecular Pain 1, 2005, 16; McKemy D.D., Nature 416, 2002, 52-58; Peier A.M., Cell 108, 2002, 705-715; Dhaka A., Annu. Rev. Neurosci. 29, 2006, 135-161). Durch Applikation von Menthol wird TRPM8 aktiviert, wodurch ein Ca²⁺⁻Einstrom in die Kälte-sensitiven Neuronen bewirkt wird. Das dadurch erzeugte elektrische Signal wird schließlich als Kältegefühl wahrgenommen. Überhöhte Menthol-Konzentrationen führen zu Irritation und einer anästhetischen Wirkung. Darüber hinaus sind in verschiedenen Publikationen Mentholderivate mit ähnlicher Wirkung beschrieben worden (British Patent 1971#1315761; Watson H.R., J. Soc. Cosmet. Chem. 29, 1978, 185-200; Furrer S.M., Chem. Percept. 1, 2008, 119-126). Es gibt auch vereinzelte, strukturell mit Menthol nicht verwandte Verbindungen, die eine signifikante TRPM8 Modulation bewirken, wie z. B. Icilin (Wei E.T., J. Pharm. Pharmacol. 35, 1983, 110-112; WO 2004/026840), WS-23 oder in der Patentanmeldung WO 2007/019719 aufgeführte Verbindungen.

Weitere Wirkungen von Substanzen, die den TRPM8 Rezeptor bzw. seine lnsektenanaloga modulieren, sind eine Repellentwirkung auf Insekten (WO 2002/015692; WO 2004/000023, US 2004/0028714), sowie Aktivität in der Antitumortherapie (z.B. eine Beeinflussung von Prostatatumoren), Aktivität bei der Behandlung von inflammatorischen Schmerzen / Hyperalgesie und eine Wirkung als TRPM8 Antagonisten in der Therapie von Blasensyndrom bzw. überaktiver Blase (Beck B. Cell Calcium, 41, 2007, 285-294; Levine J.D. Biochim. Biophys. Acta, Mol. Basis Dis. 1772, 2007, 989-1003; Mukerji G., BMC Urology 6, 2006, 6; US 2003/0207904; US 2005/6893626, Dissertation Behrendt H.J. 2004, Universität Bochum; Lashinger E.S.R. Am. J. Physiol. Renal Physiol. Am J Physiol Renal Physiol. 2008 Jun 18. [Epub ahead of print]; PMID: 18562636).

Viele der bisher gefundenen Modulatoren von TRPM8 weisen jedoch Mängel in Bezug auf Wirkstärke, Wirkdauer, Haut-/Schleimhautreizung, Geruch, Geschmack, Löslichkeit und /oder Flüchtigkeit auf.

### Kurzfassung der Erfindung

Aufgabe der vorliegenden Erfindung war es daher, neue Substanzen zu identifizieren, die zu einer Modulation des TRPM8 Rezeptors führen, welche als Alternativen zu den bisher bekannten Modulatoren einsetzbar sind. Solche Verbindungen sollten sich insbesondere auch für Anwendungen im Bereich Kosmetik (z.B. hair care, skin care, oral care), Ernährung (feed/food), Textil, OTC-Produkte (z.B. Brandsalbe), Pharmaka (z.B. Tumorbehandlung, Blasenschwäche), Verpackungen oder als Insektizid bzw. Repellent eignen.

### Figurenbeschreibung:

Figur 1 zeigt (a) die mRNA-Sequenz bzw. (b) die davon abgeleitete Aminosäuresequenz des hTRPM8 Rezeptors gemäß Sequenzdatenbank-Eintrag NM 024080 .
Figur 2 zeigt die Vektorkarte des mit hTRPM8 kodierenden Plasmids plnd_M8, welches zur Transfektion der HEK293 Zellen verwendet wurde.

### Detaillierte Beschreibung der Erfindung:

### 1. Definition allgemeiner Begriffe:

In der Literatur gibt es verschiedene Synonyme für "TRPM8": TRPP8, LTRPC6, CMR1, MGC2849, transient receptor potential cation channel subfamily M member 8. Im Sinne der vorliegenden Erfindung sind alle Bezeichnungen mit umfasst. Mit umfasst sind auch alle funktionalen Modifikationen des Rezeptors, wie insbesondere Splicevarianten, Isoformen, wie z.B. TRPM8 CRA_a, TRPM8 CRA_b und alle analogen Rezeptoren aus verschiedenen Organismen, wie Mensch, Maus, Ratte. Die Nukleotid- bzw. Aminosäuresequenzen der verschiedenen Rezeptoren sind an sich bekannt und in Sequenzdatenbanken hinterlegt. So ist z.B. die Sequenzinformation für hTRPM8 unter der Nummer NM_024080 eingetragen.

Ein "Modulator" im Sinne der Erfindung stellt eine Verbindung dar, die als Agonist des TRPM8-Rezeptors in vivo und/oder in vitro wirken kann.

Hierin beschriebene Modulatoren können entweder nur als Antagonist oder Agonist oder sowohl als Antagonist als auch als Agonist agieren. Dabei können sich insbesondere eine agonistische oder eine antagonistische Wirkung in Abhängigkeit von der jeweiligen gewählten Modulatorkonzentration einstellen.

Ein "Agonist" ist dabei eine Verbindung, welche eine Aktivierung des TRPM8-Rezeptors vermittelt, also einen Ca²⁺ -Einstrom in die kälte-sensitiven Neuronen induziert und damit ein Kältegefühl vermittelt. Ein "Antagonist" ist dagegen eine Verbindung, welche dieser Aktivierung des TRPM8-Rezeptors entgegenwirken kann.

Die erfindungsgemäßen Mediatoren können ihre Wirkung dadurch ausüben, dass sie reversibel oder irreversibel, spezifisch oder unspezifisch an ein TRPM8 Rezeptormolekül binden. Gewöhnlich erfolgt die Bindung nicht-kovalent über ionische und/oder nichtionische, wie z.B. hydrophobe, Wechselwirkungen mit dem Rezeptormolekül. "Spezifisch" umfasst dabei sowohl ausschließliche Wechselwirkung mit einem oder mehreren verschiedenen TRPM8 Rezeptormolekülen (wie z.B. TRPM8-Molekülen verschiedenen Ursprungs oder verschiedenen Isoformen). "Unspezifisch" ist dagegen eine Wechselwirkung des Modulators mit mehreren verschiedenen Rezeptormolekülen unterschiedlicher Funktion und/oder Sequenz, wobei sich jedoch als Folge eine gewünschte agonistische (und/oder antagonistische Modulation) (wie oben beschrieben) des TRPM8 Rezeptors feststellen lässt.

### 2. Bevorzugte Ausführungsformen

Ein erster Gegenstand der Erfindung betrifft ein Verfahren zur in-vitro Modulation des Kälte-Menthol-Rezeptors TRPM8, insbesondere des humanen TRPM8 Rezeptors, wobei man den Rezeptor mit einer Verbindung der Formel 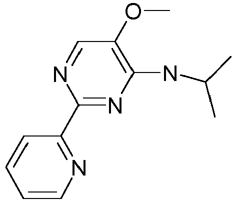 in Kontakt bringt, welche in einem zellulären Aktivitätstest, insbesondere unter Standardbedingungen, unter Verwendung von Zellen, welche den humanen TRPM8-Rezeptor rekombinant exprimieren, die Permeabilität dieser Zellen für Ca²⁺ Ionen modulieren.

Unter "Standardbedingungen" versteht man in diesem Zusammenhang einen Aktivitätstest, durchgeführt mit HEK293-Zellen, welche transformiert wurden mit humanem TRPM8 und beladen sind mit Calcium-sensitivem Farbstoff (wie z.B. Fluo-4AM, d.h. Fluo-4-Acetoxymethylester), anschließende Zugabe der Testverbindung und Nachweis der Farbänderung, wobei Versuchsdurchführung bei 37°C erfolgt; wie z.B. beschrieben in Beispiel 3, unten, oder bei Behrendt et al (2004) a.a.O).

Die erfindungsgemäß eingesetzten Modulatoren wirken dabei auf die zelluläre Ca²⁺-lonen-Permeabilität agonistisch. Insbesondere ist der Modulator eine Verbindung der folgenden Formel 3 gemäß folgender Tabelle 1 (die Verbindungen der Formeln 1-2 sowie 4-19 sind Referenzbeispiele),

**Tabelle 1: Erfindungsgemäße Modulatoren und Referenzbeispiele**

| | |
|---|---|
| 1 | 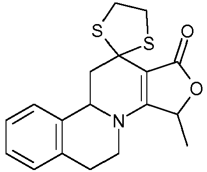 |
| 2 | 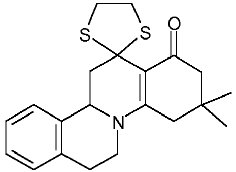 |
| 3 | 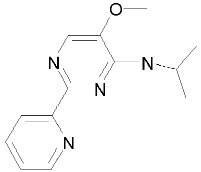 |
| 4 | 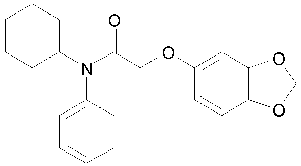 |
| 5 | 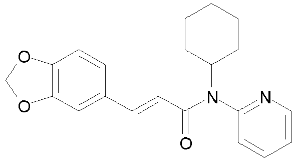 |
| 6 | 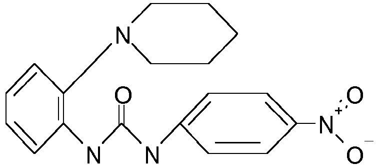 |
| 7 | 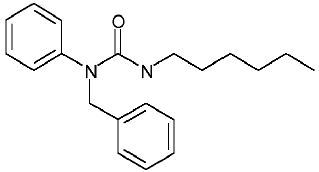 |
| 8 | 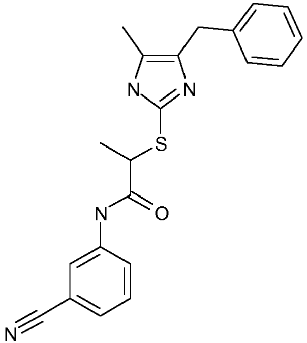 |
| 9 | 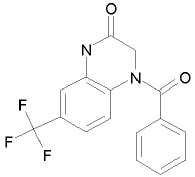 |
| 10 | 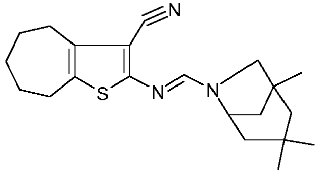 |
| 11 | 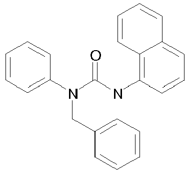 |
| 12 | 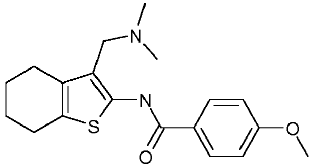 |
| 13 | 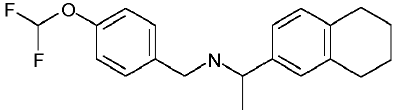 |
| 14 | 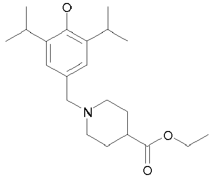 |
| 15 | 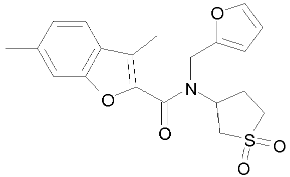 |
| 16 | 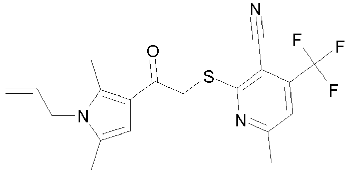 |
| 17 | 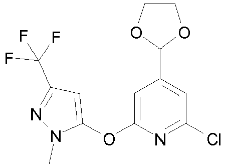 |
| 18 | 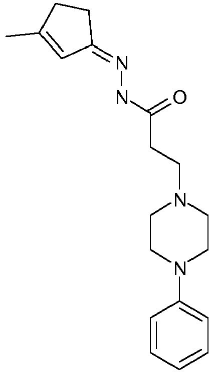 |
| 19 | 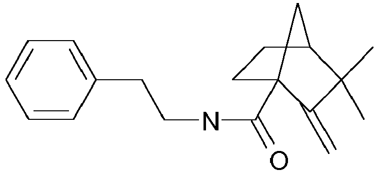 |

wobei die Verbindung in chemisch reiner oder angereicherter Form, als einzelnes Stereoisomer oder in Form von Stereoisomeren-Gemischen vorliegen kann. Weiterhin können die Verbindungen ungeladen oder in Form ihrer Salze, wie z.B. als Säureadditionssalz, vorliegen. Funktionelle Gruppen können ggf. durch äquivalente chemische Gruppen ersetzt sein; Fluoratome können so z.B. durch andere Halogenatome , wie Cl, Br oder J, ersetzt sein, Sauerstoffgruppen (wie z.B. Ethergruppen) können durch entsprechende Schwefelgruppen ersetzt sein und umgekehrt; Ketogruppen können durch entsprechende Thionylgruppen ersetzt sein. Die oben genannten Verbindungen sind an sich bekannte chemische Substanzen, die entweder käuflich erhältlich oder unter Anwendung üblicher Methoden der organischen Synthese zugänglich sind.

So sind beispielsweise bekannt:
Verbindung 1 unter CAS Nummer: 99602-94-5 (3R-cis-Form)
Verbindung 2 unter CAS Nummer: 165753-08-2
Verbindung 3 unter CAS Nummer: 338771-57-6
Verbindung 4 unter CAS Nummer: 878942-21-3
Verbindung 5 unter CAS Nummer: 748783-13-3

Die abgewandelten Formen oder Derivate werden auch als funktionale Analoga oder funktional äquivalente Verbindungen bezeichnet, wenn sie weiterhin die gewünschte biologische Aktivität (Rezeptor TRPM8 Modulation) zeigen.

Weiterhin sind besonders Derivate im Sinne der Erfindung mit umfasst, die eine Kopplung der konkret offenbarten Substanzen an feste Träger erlauben; eine große Auswahl an entsprechenden Linkern/Spacer-Gruppen ist dem Fachmann bekannt. Die Derivatisierung kann dabei vor der Kopplung an eine feste Phase oder erst durch die Kopplung erfolgen.

Ein weiterer Gegenstand der Erfindung betrifft die Verwendung eines Modulators, insbesondere Agonisten, für den TRPM8-Rezeptor, wobei der Modulator wie oben definiert ist, zur Induktion von Kältegefühl, insbesondere topisch, d.h. cutan oder oral, bei Mensch und/oder Tier. Eine "lnduktion von Kältegefühl" liegt vor, wenn die Verbindung im oben beschriebenen zellulären Aktivitätstest einen agonistischen Effekt auf hTRPM8 zeigt.

Ein weiterer Gegenstand der Erfindung betrifft die Verwendung eines Modulators für den TRPM8-Rezeptor, wobei der Modulator wie oben definiert ist, als aktiven Bestandteil eines pharmazeutischen Mittels.

Ein weiterer Gegenstand der Erfindung betrifft die Verwendung eines Modulators für den TRPM8-Rezeptor, wobei der Modulator wie oben definiert ist, zur Behandlung von Prostatakarzinomen, zur Behandlung von Blasenschwäche oder in der Schmerztherapie.

Ein weiterer Gegenstand der Erfindung betrifft die Verwendung eines Modulators für den TRPM8-Rezeptor, wobei der Modulator wie oben definiert ist, als Insekten-Repellent oder Insektizid.

Ein weiterer Gegenstand der Erfindung betrifft die Verwendung eines Modulators für den TRPM8-Rezeptor, wobei der Modulator wie oben definiert ist, zur Induktion eines Kältegefühls bei Verpackungen (z.B. aus Papier oder Kunststoff in verschiedensten Verarbeitungsformen (wie z.B. Fasern, Geweben, Formteilen), wobei sich das Kältegefühl insbesondere bei Kontakt mit dem Verpackungsmaterial bemerkbar macht. Dabei können die Substanzen in unterschiedlichster Art mit dem Verpackungsmaterial assoziiert sein: z.B. durch Spincoating, Aufdrucken, in Form von Mikroverkapselung, direkte Einarbeitung in das Verpackungsmaterial (z.B. extrudieren), kovalente Kopplung von geeigneten Derivaten der Modulatoren (über geeignete Spacer/Linker-Gruppen, mit deren Hilfe das Molekül reversibel oder irreversibel an das Verpackungsmaterial gebunden wird). Geeignete Arbeitsweisen sind dem Fachmann bekannt.

Ein weiterer Gegenstand der Erfindung betrifft die Verwendung eines Modulators für den TRPM8-Rezeptor, wobei der Modulator wie oben definiert ist, zur Induktion eines Kältegefühls bei Textilien. Dabei können die Substanzen in unterschiedlichster Art mit dem Textil assoziiert sein: z.B. durch Spincoating, Aufdrucken, in Form von Mikroverkapselung, direkte Einarbeitung in das Textilmaterial (z.B. extrudieren), kovalente Kopplung von geeigneten Derivaten der Modulatoren (über geeignete Spacer/Linker-Gruppen, mit deren Hilfe das Molekül reversibel oder irreversibel an das Verpackungsmaterial gebunden wird). Geeignete Arbeitsweisen sind dem Fachmann bekannt.

Ein weiterer Gegenstand der Erfindung betrifft Stoffe per se gemäß obiger Definition zur Verwendung als Mediator, insbesondere Agonisten, des TRPM8 Rezeptors in einem Medikament.
Ein weiterer Gegenstand der Erfindung betrifft Mittel, enthaltend wenigstens eine Verbindung nach obiger Definition. Insbesondere sind solche Mittel ausgewählt unter
a) pharmazeutischen Mitteln, wie Antitumormittel, Mittel zur Behandlung von Erkrankungen der Blase, Schmerzmittel;
b) Nahrungsmitteln, wie Eis, Mousse, Creme, Getränke, Süßwaren,
c) Mundpflegemitteln, wie Zahnpasta, Mundwasser, Kaugummi, Atemerfrischungsmittel
d) Haut-, oder Haarpflegemitteln, wie Sonnencreme, Sonnenbrandcreme, Lotionen, Shampoos, Pflaster, Mundwasser, Lotionen, Rasiercreme, Conditioner, Gesichtsreiniger, Seifen, Badeöle und Badeschäume, Antitranspirationsmittel, desodorierende Mittel,
e) lnsektenrepellents, Insektiziden.

Derartige Mittel enthalten neben für das jeweilige Mittel jeweils üblichen Bestandteilen eine wirksame Menge wenigstens eines erfindungsgemäßen Modulators. "Wirksam" bedeutet in diesem Zusammenhang eine Konzentration des Modulators, die ausreicht, um bei Applikation des Mittels (z.B. Auftragung auf die Haut) den gewünschten Effekt, wie z.B. pharmakologischen Effekt, oder sensorischen Effekt wie z.B. den olfaktorischen Kälteeffekt zu vermitteln.

Gegebenenfalls können die erfindungsgemäßen Verbindungen mit weiteren bekannten Wirkstoffen, insbesondere auch solchen mit vergleichbarer Wirkung, kombiniert werden. Beispielsweise können diese mit bekannten kühlenden Verbindungen, wie z.B. Menthol, Menthon, N-Ethyl-p-menthancarboxamid (WS-3), N-2,3-Trimethyl-2-isopropylbutanamid (WS-23), Menthyllactat (Frescolat® ML), Menthonglycerinacetal (Frescolat® MGA), Mono-menthylsuccinat (Physcool®), Mono-menthyl glutarat, O-Menthyl Glycerin, Menthyl-N,N-dimethylsuccinamat kombiniert werden.

Gegenstand der Erfindung sind weiterhin Textilprodukte, wie z.B. Hemden, Hosen, Socken, Handtücher, ausgerüstet (insbesondere oberflächlich) mit wenigstens einer Verbindung nach obiger Definition.

Gegenstand der Erfindung sind weiterhin Verpackungsmaterialien, welche mit wenigstens einer Verbindung nach obiger Definition assoziiert sind.

Die Erfindung wird nun anhand folgender nicht-limitierender Ausführungsbeispiele beschrieben.

### Experimenteller Teil:

### Beispiel 1 - Klonierung von humanem TRPM8

Ausgangspunkt für die Klonierung des humanen TRPM8 Rezeptors ist eine LnCaP cDNA-Bank. Diese ist beispielsweise kommerziell erhältlich (z. B. Firma BioChain, Hayward, USA) oder aus der androgensensitiven humanen Prostata-Adenokarzinomzelllinie LnCaP (z.B. ATCC, CRL1740 oder ECACC, 89110211) unter Verwendung von Standardkits herstellbar.

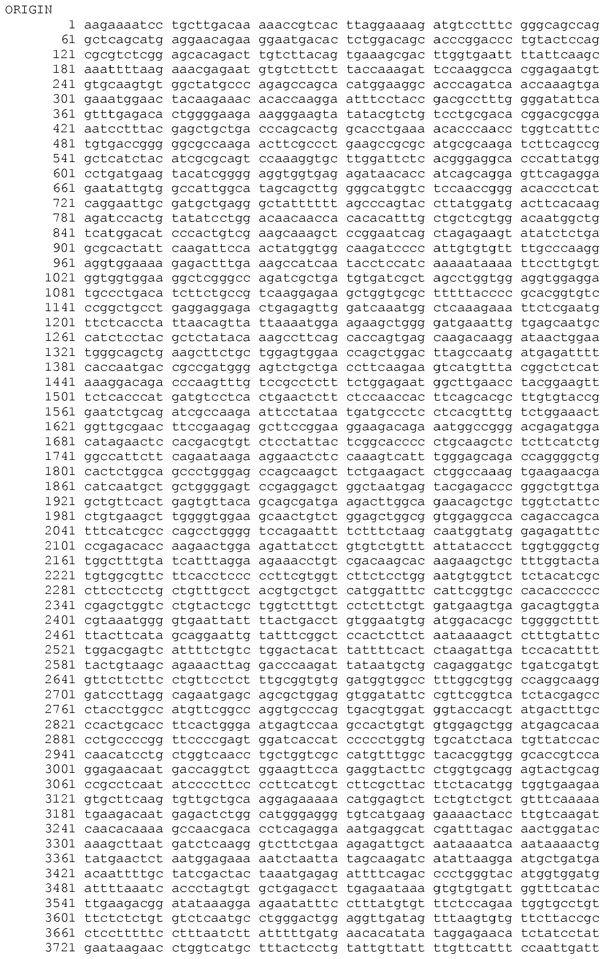

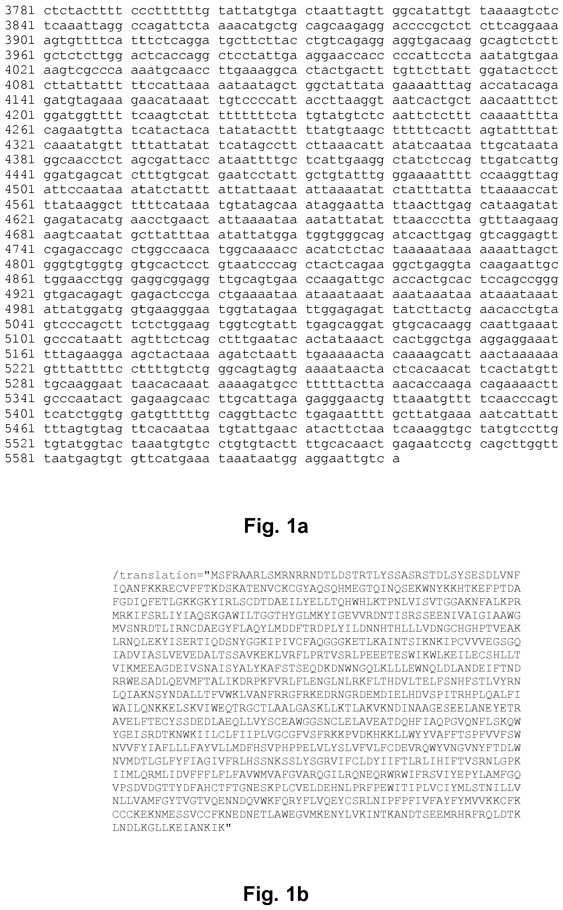

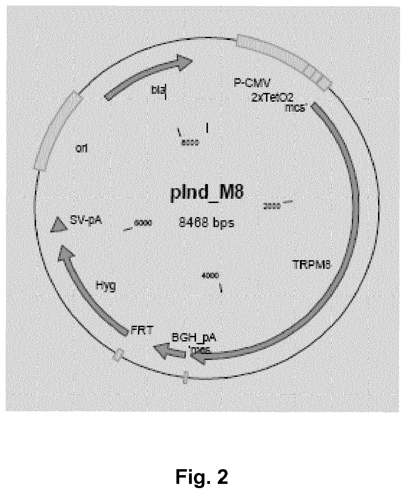

Die kodierende TRPM8 Sequenz (vgl Fig.1A; sowie **http://www.ncbi.nlm.nih.gov/entrez/viewer.fcgi?db=nuccore&id=109689694)** kann unter Verwendung von Standardmethoden PCR-amplifiziert und kloniert werden. Das so isolierte humane TRPM8 Gen wurde zur Herstellung des Plasmids plnd_M8 verwendet, dessen Konstruktion durch die Plasmidkarte gemäß Figur 2 veranschaulicht wird.

Alternativ dazu kann das TRPM8 Gen auch synthetisch hergestellt werden.

### Beispiel 2 - Generierung der HEK293-Testzellen

Als Testzellsystem wurde mit der humanen TRPM8 DNA (vgl obiges Plasmid plnd-M8) stabil transfizierte HEK293 Zelllinie hergestellt. Bevorzugt wird dabei HEK293 die über das eingebrachte Plasmid die Möglichkeit zur Induktion der TRPM8-Expression mittels Tetrazyklin bietet.

Methoden zur Herstellung geeigneter Testzellsysteme sind dem Fachmann bekannt. So kann man die Herstellung der erfindungsgemäß verwendeten Zellen den Angaben in Behrendt H.J. et al.,, Br. J. Pharmacol. 141, 2004, 737-745 oder der Dissertation von Behrendt "Vergleichende funktionale Untersuchungen des Hitze-Capsaicin-Rezeptors (TRPV1) und des Kälte-Menthol-Rezeptors (TRPM8) in rekombinanten und nativen Zellssystemen", zugänglich unter http://www-brs.ub.ruhr-uni-bochum.de/netahtml/HSS/Diss/BehrendtHansJoerg/diss.pdf entnehmen. Auf die Offenbarung dieser Druckschriften wird ausdrücklich Bezug genommen.

### Beispiel 3- Assay auf TRPM8 Modulatoren

Es wird ein Test, vergleichbar mit dem bereits in der Literatur beschrieben Test von Behrendt H.J. et al., Br. J. Pharmacol. 141, 2004, 737-745, durchgeführt. Die Agonisierung bzw. Antagonisierung des Rezeptors kann mittels eines Ca²⁺-sensitiven Farbstoffs (z.B. FURA, Fluo-4 etc.) quantifiziert werden. Agonisten bewirken alleine eine Zunahme des Ca²⁺-Signals; Antagonisten bewirken in Gegenwart von z.B. Menthol eine Reduzierung des Ca²⁺-Signals (jeweils detektiert über den Farbstoff Fluo-4, der durch Ca²⁺ andere Fluoreszenzeigenschaften hat).

### a) Testablauf:

Zunächst wird in an sich bekannter Weise in Zellkulturflaschen eine frische Kultur transformierter HEK-Zellen hergestellt. Die Testzellen HEK293-TRPM8 werden mittels Trypsin von den Zellkulturflaschen abgelöst und 40.000 Zellen/well werden mit 100 µl Medium in 96-Lochplatten (Greiner # 655948 Poly-D-Lysin-beschichtet) ausgesät. Zur Induktion des Rezeptors TRPM8 wird dem Wachstumsmedium Tetrazyklin beigemischt (DMEM/HG, 10% FCS tetrazyklinfrei, 4 mM L-Glutamin, 15 µg/ml Blasticidin, 100 µg/ml Hygromycin B, 1 µg/ml Tetrazyklin). Am darauffolgenden Tag werden die Zellen mit Fluo-4AM Farbstoff beladen und der Test wird durchgeführt. Dazu wird wie folgt vorgegangen:
- Zugabe von je 100 µl/well Färbelösung Ca-4 Kit (RB 141, Molecular Devices) zu je 100 µl Medium (DMEM/HG, 10% FCS tetrazyklinfrei, 4 mM L-Glutamin, 15 µg/ml Blasticidin, 100 µg/ml Hygromycin B, 1 µg/ml Tetrazyklin)
- Inkubation im Brutschrank, 30 Minuten / 37°C / 5% CO₂, 30 Minuten / RT
- Vorbereitung der Testsubstanzen (unterschiedliche Konzentrationen in 200 µl HBSS-Puffer), sowie von Positivkontrollen (unterschiedliche Konzentrationen von Menthol, Icilin bzw. lonomycin in 200 µl HBSS-Puffer) und Negativkontrollen (nur 200 µl HBSS-Puffer)
- Zugabe der Testsubstanzen in Mengen von 50 µl/well und Messung der Fluoreszenzänderung (z.B. im Assaygerät FLIPR, Molecular Devices oder NovoStar, BMG) bei 485 nm Anregung, 520 nm Emission, und Auswertung der Wirkstärke der verschiedenen Substanzen/Konzentrationen und Bestimmung der EC50-Werte

Die Testsubstanzen werden in Triplikaten in Konzentrationen von 0,1 - 200 µM im Assay eingesetzt. Normalerweise werden die Verbindungen in DMSO-Lösungen bereitgehalten und für den Assay auf eine maximale DMSO-Konzentration von 2% herunterverdünnt.

### b) Testergebnis

Die ermittelten EC50-Werte erfindungsgemäßer Modulatoren sind in folgender Tabelle 2 zusammengefasst

**Tabelle 2: Aktivität von Testsubstanzen auf den human Rezeptor TRPM8**

| **#** | **Activität TRPM8 EC50** |
|---|---|
| 1 | 0,4 |
| 2 | 2 |
| 3 | 2 |
| 4 | 2,5 |
| 5 | 2,5 |
| 6 | 3,5 |
| 7 | 4 |
| 8 | 5 |
| 9 | 10 |
| 10 | 10 |
| 11 | 10 |
| 12 | 10 |
| 13 | 10 |
| 14 | 20 |
| 15 | 20 |
| 16 | 25 |
| 17 | 50 |
| 18 | 100 |
| 19 | 100 |

Die Auswertung zeigt überraschenderweise, dass erfindungsgemäß erstmalig Agonisten von TRPM8 bereitgestellt werden konnten, welche sich strukturell von bisher bekannten Agonisten, wie (-) Menthol, Icilin und anderen von Behrendt H.J. et al.,in Br. J. Pharmacol. 141, 2004, 737-745 (vgl dortige Tabelle 1) beschriebenen Modulatoren signifikant unterscheiden und zudem teilweise bessere Aktivitäten als (-) Menthol zeigen, bzw. vergleichbar stark wirken wie Icilin.

### Beispiel 4 - Herstellung von Mundwasser

Ein Mundwasser folgender Zusammensetzung wird hergestellt:

| | |
|---|---|
| Ethanol 95% | 177mL |
| Sorbit 70% | 250 g |
| TRPM8 Agonist gemäß von Tab.2 als 1% Lösung im Ethanol | 50mL |
| Pfefferminzöl, | 0.30 g |
| Methylsalicylat | 0.64 g |
| Eucalyptol | 0.922 g |
| Thymol | 0.639 g |
| Benzoesäure | 1.50 g |
| Pluronic ® F127 nichtionisches Tensid | 5.00 g |
| Natrium-Saccharin | 0.60 g |
| Natriumcitrat | 0.30 g |
| Zitronensäure | 0.10 g |
| Wasser q.s. 1 Liter | |

Zur Herstellung eines Mundwassers werden die oben beschriebenen Komponenten in den angegebenen Mengen miteinander vermischt.

Auf die Offenbarung der hierin zitierten Literaturstellen wird hiermit ausdrücklich Bezug genommen.

## Patentansprüche

1. Verfahren zur in-vitro Modulation des Kälte-Menthol-Rezeptors TRPM8, wobei man den Rezeptor mit einer Verbindung der Formel 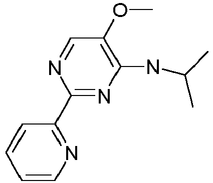 in Kontakt bringt, welche in einem zellulären Aktivitätstest unter Verwendung von Zellen, welche den humanen TRPM8-Rezeptor rekombinant exprimieren, die Permeabilität dieser Zellen für Ca²⁺ Ionen moduliert.

2. Verfahren nach Anspruch 1, wobei der Modulator auf die zelluläre Ca²⁺-lonen-Permeabilität agonistisch wirkt.

3. Verwendung eines Modulators für den TRPM8-Rezeptor, wobei der Modulator gemäß Anspruch 1 definiert ist, zur Induktion von Kältegefühl bei Mensch und/oder Tier.

4. Verwendung eines Modulators für den TRPM8-Rezeptor, wobei der Modulator gemäß Anspruch 1 definiert ist, als aktiven Bestandteil eines pharmazeutischen Mittels.

5. Modulator für den TRPM8-Rezeptor, wobei der Modulator gemäß Anspruch 1 definiert ist, zur Verwendung bei der Behandlung von Prostatakarzinomen, von Blasenschwäche oder in der Schmerztherapie.

6. Verwendung eines Modulators für den TRPM8-Rezeptor, wobei der Modulator gemäß Anspruch 1 definiert ist, als Insekten-Repellent oder Insektizid.

7. Verwendung eines Modulators für den TRPM8-Rezeptor, wobei der Modulator gemäß Anspruch 1 definiert ist, zur Induktion eines Kältegefühls in einer Verpackung oder einem Textil.

8. Stoff gemäß der Definition nach Anspruch 1 zur Verwendung als Medikament.

9. Mittel enthaltend im Gemisch mit üblichen Bestandteilen eine Verbindung nach Anspruch 1.

10. Mittel nach Anspruch 9, ausgewählt unter
a) pharmazeutischen Mitteln, wie Antitumormittel, Mittel zur Behandlung von Erkrankungen der Blase, Schmerzmittel;
b) Nahrungsmitteln, wie Eis, Mousse, Creme, Getränke, Süßwaren,
c) Mundpflegemitteln, wie Zahnpasta, Mundwasser, Kaugummi,
d) Haut- oder Haarpflegemitteln, wie Sonnencreme, Sonnenbrandcreme, Lotionen, Shampoos, Pflaster,
e) Insektenrepellents, Insektiziden.

11. Textilprodukte, wie Hemden, Hosen, Socken, Handtücher, ausgerüstet mit einer Verbindung nach Anspruch 1.

12. Verpackungsmaterial, welches mit einer Verbindung nach Anspruch 1 assoziiert ist.

## Claims

1. A method for the in-vitro modulation of the cold menthol receptor TRPM8, where the receptor is brought into contact with a compound of the formula 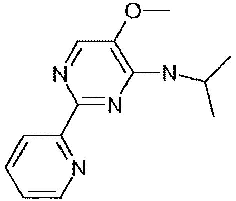 which, in a cellular activity test using cells which recombinantly express the human TRPM8 receptor, modulates the permeability of these cells for Ca²⁺ ions.

2. The method according to claim 1, where the modulator has an agonistic effect on the cellular Ca²⁺ ion permeability.

3. The use of a modulator for the TRPM8 receptor, where the modulator is defined according to claim 1, for inducing a sensation of coldness in humans and/or animals.

4. The use of a modulator for the TRPM8 receptor, where the modulator is defined according to claim 1, as active constituent of a pharmaceutical composition.

5. A modulator for the TRPM8 receptor, where the modulator is defined according to claim 1, for use in the treatment of prostate carcinomas, of bladder weakness or in pain therapy.

6. The use of a modulator for the TRPM8 receptor, where the modulator is defined according to claim 1, as insect repellent or insecticide.

7. The use of a modulator for the TRPM8 receptor, where the modulator is defined according to claim 1, for inducing a sensation of coldness in a packaging or a textile.

8. A substance according to the definition according to claim 1 for use as medicament.

9. A composition comprising, in a mixture with customary constituents, a compound according to claim 1.

10. The composition according to claim 9, selected from
a) pharmaceutical compositions, such as antitumor agents, agents for the treatment of diseases of the bladder, painkillers;
b) foods, such as ice cream, mousse, cream, beverages, confectionery,
c) mouthcare compositions, such as toothpaste, mouthwash, chewing gum,
d) skincare or haircare compositions, such as suncream, sunburn cream, lotions, shampoos, plasters,
e) insect repellents, insecticides.

11. A textile product such as a shirt, trousers, socks, a towel, finished with a compound according to claim 1.

12. A packaging material which is associated with a compound according to claim 1.

## Revendications

1. Procédé de modulation in vitro du récepteur au froid et au menthol TRPM8, selon lequel le récepteur est mis en contact avec un composé de la formule 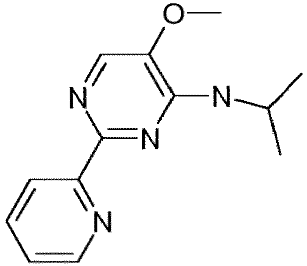 qui, lors d'un test d'activité cellulaire utilisant des cellules qui expriment de manière recombinante le récepteur TRPM8 humain, module la perméabilité de ces cellules pour les ions Ca²⁺.

2. Procédé selon la revendication 1, selon lequel le modulateur a un effet agonistique sur la perméabilité cellulaire pour les ions Ca²⁺.

3. Utilisation d'un modulateur pour le récepteur TRPM8, le modulateur étant défini selon la revendication 1, pour l'induction d'une sensation de froid chez un homme et/ou un animal.

4. Utilisation d'un modulateur pour le récepteur TRPM8, le modulateur étant défini selon la revendication 1, en tant que constituant actif d'un agent pharmaceutique.

5. Modulateur pour le récepteur TRPM8, le modulateur étant défini selon la revendication 1, destiné à une utilisation lors du traitement de cancers de la prostate, d'une faiblesse de la vessie ou dans le traitement de la douleur.

6. Utilisation d'un modulateur pour le récepteur TRPM8, le modulateur étant défini selon la revendication 1, en tant qu'agent répulsif contre les insectes ou insecticide.

7. Utilisation d'un modulateur pour le récepteur TRPM8, le modulateur étant défini selon la revendication 1, pour l'induction d'une sensation de froid dans un emballage ou un textile.

8. Substance selon la définition de la revendication 1, destinée à une utilisation en tant que médicament.

9. Agent contenant en mélange avec des constituants usuels un composé selon la revendication 1.

10. Agent selon la revendication 9, choisi parmi :
a) les agents pharmaceutiques, tels que les agents anti-tumoraux, les agents pour le traitement de maladies de la vessie, les analgésiques ;
b) les produits alimentaires, tels que les glaces, les mousses, les crèmes, les boissons, les confiseries ;
c) les agents pour l'hygiène buccale, tels que les dentifrices, les bains de bouche, les gommes à mâcher,
d) les agents de soin de la peau ou des cheveux, tels que les crèmes solaires, les crèmes pour les coups de soleil, les lotions, les shampoings, les pansements,
e) les agents répulsifs contre les insectes, les insecticides.

11. Produits textiles, tels que des chemises, des pantalons, des chaussettes, des serviettes, traités avec un composé selon la revendication 1.

12. Matériau d'emballage, qui est associé avec un composé selon la revendication 1.
